Europäisches Patentamt

European Patent Office

Office européen des brevets

(11) Numéro de publication: **0 402 968 B1**

# FASCICULE DE BREVET EUROPEEN

(45) Date de publication de fascicule du brevet: **12.10.94**

(21) Numéro de dépôt: **90201145.1**

(22) Date de dépôt: **07.05.90**

(51) Int. Cl.⁵: **A61B 8/06**, G01S 15/89, G01S 15/52

(54) **Dispositif de mesure de la vitesse d'écoulements sanguins.**

(30) Priorité: **12.05.89 FR 8906289**

(43) Date de publication de la demande:
**19.12.90 Bulletin 90/51**

(45) Mention de la délivrance du brevet:
**12.10.94 Bulletin 94/41**

(84) Etats contractants désignés:
**BE DE FR GB SE**

(56) Documents cités:
**EP-A- 0 161 956**
**EP-A- 0 225 667**
**EP-A- 0 242 914**
**EP-A- 0 298 569**
**US-A- 3 882 444**

(73) Titulaire: **LABORATOIRES D'ELECTRONIOUE PHILIPS**
**3, Avenue Descartes**
**F-94450 Limeil-Brévannes (FR)**

(84) Etats contractants désignés:
**FR**

(73) Titulaire: **N.V. Philips' Gloeilampenfabrieken**
**Groenewoudseweg 1**
**NL-5621 BA Eindhoven (NL)**

(84) Etats contractants désignés:
**BE DE GB SE**

(72) Inventeur: **Bonnefous, Odile**
**Societé Civile S.P.I.D.,**
**156, Boulevard Haussmann**
**F-75008 Paris (FR)**

(74) Mandataire: **Pyronnet, Jacques et al**
**Société Civile S.P.I.D.**
**156, Boulevard Haussmann**
**F-75008 Paris (FR)**

**Description**

La présente invention concerne un dispositif de mesure de la vitesse d'écoulements sanguins à partir d'une séquence de N signaux échographiques successifs, comprenant un éliminateur d'échos fixes à M points, suivi d'une unité de mesure de ladite vitesse par corrélation-sommation-interpolation des N-M+1 signaux indépendants issus dudit éliminateur d'échos fixes.

L'invention trouve une application particulièrement avantageuse dans le domaine général de l'exploration échographique d'écoulements sanguins dans les vaisseaux, et plus particulièrement dans celui de la visualisation de tels écoulements.

On connaît de la demande de brevet européen n° 0 225 667 un dispositif de mesure conforme au préambule dans lequel ladite unité de mesure est constituée d'une mémoire dans laquelle sont stockés lesdits N-M+1 signaux indépendants, d'un corrélateur fournissant N-M fonctions d'intercorrélation des N-M+1 signaux mémorisés, d'un additionneur donnant la fonction d'intercorrélation moyenne, et d'un circuit d'interpolation, qui permet de déterminer le maximum de la fonction d'intercorrélation moyenne, appelée aussi pic de corrélation, dont la position est directement liée à la vitesse d'écoulements recherchée. On peut, en effet, rappeler que le dispositif connu de l'état de la technique utilise le fait que les signaux ultrasonores successifs rétrodiffusés par une cible en mouvement, lorsque l'émission est récurrente à la période de récurrence T, sont reliés par l'équation suivante :

$$S_{n+1}(t) = S_n(t-\tau)$$

Ceci signifie que le signal $n+1$ est la réplique du signal $n$ précédent à un décalage temporel $\tau$ près. Ce dernier représente le temps supplémentaire nécessaire à l'onde ultrasonore pour parcourir le trajet transducteur-cible-transducteur, d'un tir à l'autre. Autrement dit :

$$\tau = 2VT/C$$

où V est la vitesse de la cible et C la vitesse du son. On voit qu'une mesure de $\tau$ permet de mesurer la vitesse V cherchée.

La fonction d'intercorrélation entre $S_n(t)$ et $S_{n+1}(t)$ définie, sur une fenêtre de largeur W, par la relation :

$$C_{n,n+1}(to,u) = \int_{to}^{to+W} S_{n+1}(t+u) \; S_n(t)dt$$

vérifie :

$$C_{n,n+1}(to,u) = C_{nn}(to,u-\tau)$$

Le temps to est lié à la profondeur d'exploration z par $to = 2z/C$.

La fonction $C_{nn}(to,u)$ est une fonction d'autocorrélation, et, de ce fait, est maximale pour $u = o$.

Ainsi, une mesure du décalage temporel $\tau$, et donc de la vitesse V, peut se faire en cherchant pour quel paramètre u la fonction $C_{n,n+1}(to,u)$ est maximale. Si ce maximum est obtenu pour la valeur uo de u, on en déduit $\tau$ en utilisant l'égalité $uo = \tau$ et $V = uoC/2T$.

Le dispositif décrit dans la demande de brevet européen mentionnée plus haut a apporté à la mesure de la vitesse des écoulements sanguins, et à leur visualisation, une contribution décisive. Il faut toutefois remarquer que la précision du résultat obtenu dépend du nombre de fonctions d'intercorrélation dont on fait la moyenne, c'est-à-dire N-M pour le dispositif connu. Dans le but de réduire la variance de la mesure, il y a donc avantage à augmenter le nombre N de signaux à traiter simultanément, ou à diminuer le nombre de points M de l'éliminateur d'échos fixes.

En pratique, il n'est guère possible de réduire sensiblement M au-dessous de 3 ou 4. On sait en effet que l'utilisation d'un dispositif d'élimination d'échos fixes est indispensable, avant toute estimation de profil de vitesses, du fait de la grande réflectivité des parois des vaisseaux qui peut dépasser de 40 dB la réflectivité du sang (globules blancs). L'éliminateur d'échos fixes le plus simple est celui dit à 2 points, il est composé d'une ligne retardée d'une période de récurrence en parallèle sur une ligne de retard nul. Des coefficients de pondération, respectivement ±1, sont affectés à ces lignes qui, après pondération, sont additionnées par un additionneur. Ce filtre connu réalise donc la différence entre deux lignes échographiques consécutives ce qui, par principe, conduit à une diminution quasi complète des échos produits par les tissus fixes. Cependant, cette technique présente l'inconvénient majeur d'atténuer également les signaux correspondant aux faibles vitesses d'écoulement. A titre d'exemple, on peut montrer que la réponse du filtre ci-dessus en fonction de la vitesse d'écoulement est telle que, pour une fréquence de récurrence de 5kHz et une fréquence d'émission de 5 MHz, un signal correspondant à v = 5 cm/s est atténué de 30 dB. Ceci rend difficile, voire impossible, la mesure des vitesses d'écoulement là ou elles sont les plus faibles, c'est-à-dire près des parois des vaisseaux. Or, la connaissance de ces vitesses est très importante pour l'étude et le diagnostic clinique des artères, par exemple. C'est pourquoi il est préférable d'utiliser un éliminateur d'échos à au moins 3 points comme celui décrit dans la demande de brevet européen n° 0 298 569 qui permet de s'affranchir complètement des échos produits par les tissus fixes sans pour autant réduire de façon excessive les signaux provenant des écoulements de faible vitesse.

On en conclut que la seule façon de pouvoir améliorer la précision de la mesure serait d'augmenter le nombre N de signaux successifs utilisés pour obtenir une estimée de la vitesse. Toutefois, il est clair que cette solution a pour conséquence immédiate de diminuer la cadence de mesure, ou la cadence d'image dans le cas d'une visualisation, celle-ci étant donnée par 1/NT.

Aussi, le problème technique à résoudre par l'objet de la présente invention est de réaliser un dispositif de mesure conforme au préambule qui permettrait d'obtenir une meilleure précision de mesure sans en altérer la cadence, ou, ce qui revient au même, d'augmenter la cadence de mesure sans diminuer la précision.

La solution au problème technique posé consiste, selon la présente invention, en ce que ladite unité de mesure comporte, d'une part, $N_F$ voies parallèles de traitement constituées, chacune, d'un filtre, les $N_F$ filtres étant complètement décorrélés entre eux, d'une mémoire de stockage des N-M+1 signaux filtrés, et d'un corrélateur fournissant N-M fonctions d'intercorrélation, et, d'autre part, un additionneur qui réalise la moyenne des $N_F(N-M)$ fonctions d'intercorrélation obtenues, un circuit d'interpolation appliqué à la fonction d'intercorrélation moyenne fournissant une estimée de la vitesse recherchée. L'effet technique essentiel qui se trouve à la base de l'invention réside donc dans la multiplication par un facteur $N_F$ du nombre de fonctions d'intercorrélation indépendantes à partir desquelles est calculée la fonction d'intercorrélation moyenne. Ceci est rendu possible par le fait que les opérations de filtrage conservent les relations temporelles entre les signaux et conduisent, par conséquent, aux mêmes résultats en corrélation. L'indépendance des fonctions d'intercorrélation obtenues par des voies parallèles de traitement différentes est assurée par l'absence de corrélation entre les $N_F$ filtres. Ainsi, pour la même fréquence de mesure 1/NT, on obtient une estimée de vitesse dont la précision est améliorée d'un facteur $\sqrt{N_F}$. Inversement, si 1/NoT est la cadence de mesure du dispositif connu décrit plus haut donnant la même précision que le dispositif selon l'invention, la relation entre N et No est donnée par :

$$N_F(N-M) = NoM$$

soit :

$$N = (No-M)/N_F + M$$

avec No = 15, M = 3 et $N_F$ = 6, on a N = 5, ce qui correspond à un gain d'un facteur 3 en cadence de mesure. On remarque, cependant, que lorsqu'on augmente le nombre $N_F$ de voies parallèles de traitement, on obtient une valeur limite pour N égale à M.

Le choix des $N_F$ filtres est guidé par l'idée de réaliser artificiellement $N_F$ transducteurs piézoélectriques différents en appliquant des filtrages différents à un seul transducteur, sans perdre de vue le souci de ne pas dégrader la résolution du signal. Aussi, il est prévu que, dans un mode de réalisation particulier de l'invention, Fe étant la fréquence d'échantillonnage du dispositif, chaque filtre est défini sur l'intervalle ]0,Fe/2] par une réponse en module égale à 1 et une phase aléatoire, et par une réponse nulle pour la fréquence zéro. Chacun des $N_F$ filtres est donc caractérisé par sa répartition aléatoire de phase. On peut également remarquer que ce type de filtres préserve la bande de fréquence des signaux.

Enfin, de façon connue, on peut utiliser un mode particulier de mise en oeuvre utilisant la corrélation dite "1 bit", et dans lequel chaque mémoire de stockage ne mémorise que le signe des N-M+1 signaux issus du filtre correspondant. Dans ce cas, le pic de la fonction d'intercorrélation est de forme triangulaire isocèle. La connaissance de cette forme permet à partir du point le plus haut et de ses deux voisins de reconstituer complètement, par interpolation linéaire, le pic de corrélation et donc de déterminer avec précision l'emplacement uo de son maximum.

La description qui va suivre en regard des dessins annexés, donnés à titre d'exemples non limitatifs, fera bien comprendre en quoi consiste l'invention et comment elle peut être réalisée.

La figure 1 est le schéma d'un dispositif de mesure selon l'invention.

Les figures 2a et 2b donnent la réponse en fréquence et la phase de filtres utilisés dans le dispositif de la figure 1.

La figure 3 donne quelques exemples de réponses temporelles de filtres qui ont une réponse en fréquence conforme à celle de la figure 2.

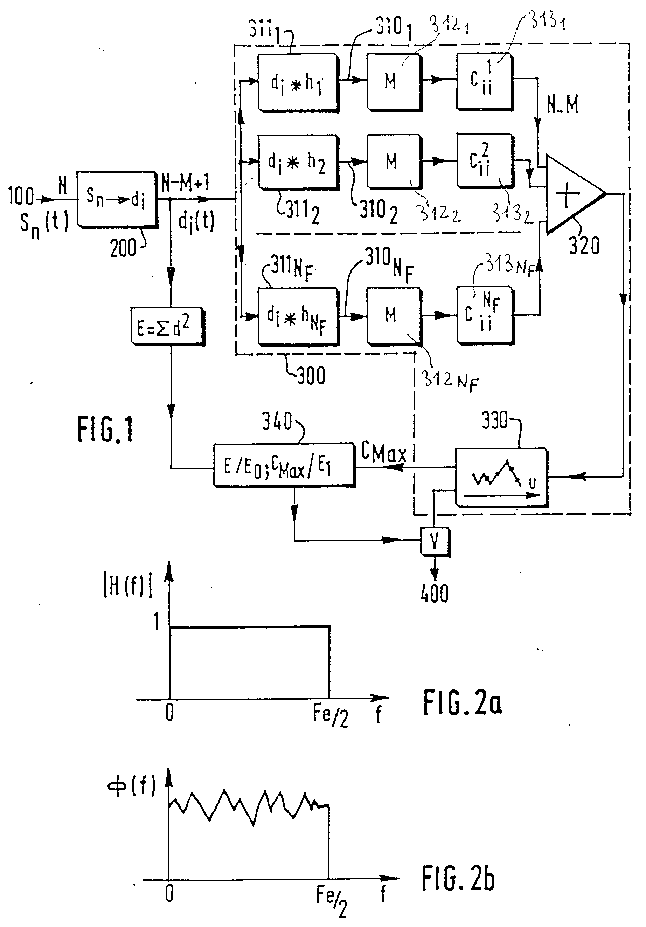

La figure 1 montre, sous forme schématique, un dispositif de mesure de la vitesse d'écoulements sanguins à partir d'une séquence de N signaux échographiques successifs $S_n(t)$, n variant par pas de 1 de 1 à N. Ces signaux échographiques proviennent d'une unité 100 d'émission-réception, non représentée, mais comprenant, de façon classique, un transducteur piézoélectrique qui convertit en trains périodiques d'impulsions ultrasonores de période de récurrence T les signaux électriques d'excitation qu'il reçoit d'un étage d'émission. Un étage de réception fournit à sa sortie les signaux échographiques $S_n(t)$, échantillonnés à la fréquence d'échantillonnage Fe, renvoyés vers le transducteur piézoélectrique par le milieu soumis à observation. Le dispositif de mesure de vitesse de la figure 1 comporte un éliminateur 200 d'échos fixes à M points qui, de la séquence des N signaux échographiques $S_n(t)$, donne une séquence de N-M+1 signaux $d_i(t)$ indépendants et exempts des composantes de forte amplitude provenant des parois fixes des vaisseaux. Comme indiqué plus haut, un exemple d'éliminateur d'échos fixes à M = 3 points qui pourrait convenir au dispositif selon l'invention est donné dans la demande de brevet européen n° 0 298 569.

Ainsi que le montre la figure 1, l'éliminateur 200 d'échos fixes est suivi d'une unité de mesure 300 de la vitesse V de l'écoulement sanguin considéré, par corrélation-sommation-interpolation des N-M+1 signaux indépendants $d_i(t)$ issus de l'éliminateur d'échos fixes, conformément à l'enseignement général de la demande de brevet européen n° 0 225 667. Comme on peut le voir à la figure 1, l'unité de mesure 300 comporte, en premier lieu, $N_F$ voies parallèles de traitement notées 310j, avec $j = 1,2,...,N_F$. Chacune de ces voies de traitement comprend un filtre 311j dont la réponse H(f) en fonction de la fréquence f est schématisée sur les figures 2a et 2b : le module $|H(f)|$ vaut 0 pour f = 0 et 1 de la fréquence 0 à la fréquence Fe/2, Fe étant la fréquence d'échantillonnage, tandis que la phase $\phi(f)$ est, dans le même intervalle, une fonction aléatoire de la fréquence :

$$\phi(f) = \text{Ran}(f)$$

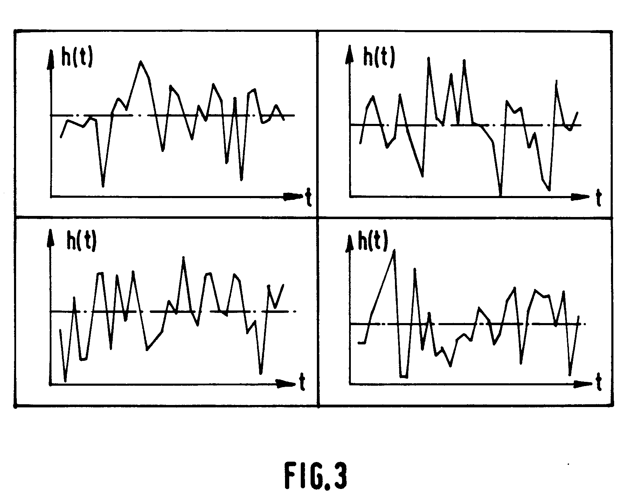

Quelques exemples de réponses temporelles h(t) de filtres de ce type sont donnés sur la figure 3. Les quatre filtres montrés sur cette figure ont la propriété d'être complètement décorrélés entre eux, ce qui assure l'entière indépendance des $N_F$ voies de traitement les unes par rapport aux autres, cette propriété étant essentielle pour assurer l'efficacité du dispositif selon l'invention. L'opération effectuée par les filtres 311j est une convolution qui, du signal entrant $d_i(t)$ donne, en sortie, le signal $d_i^j(t)$ égal à :

$$d_i^j(t) = d_i(t) \times h_j(t)$$

Ensuite, la voie 310j de traitement comporte une mémoire 312j de stockage des valeurs des N-M+1 signaux filtrés $d_i^j(t)$ servant au calcul, par un corrélateur 313j, des N-M fonctions d'intercorrélation définies par :

$$C_{ii}^j(to,u-\tau) = C_{i,i+1}^j(to,u) = \int_{to}^{to+W} d_{i+1}^j(t+u)\, d_i^j(t)dt.$$

Les signaux $d_i^j(t)$ utilisés pour déterminer les fonctions d'intercorrélation peuvent être limités aux seuls signes des signaux eux-mêmes. Cette méthode de mise en oeuvre simplifie considérablement les calculs de corrélation et la taille des mémoires 312j dans la mesure où les signaux traités n'occupe qu'un mot d'1 bit.

Le filtrage par les filtres 311j n'affecte pas le résultat des corrélations, car la convolution ne modifie pas les relations temporelles entre signaux, en d'autres termes :

4

$$\int_{to}^{to+W} d_{i+1}^{j}(t+u) \; d_{i}^{j}(t)dt = \int_{to}^{to+W} d_{i+1}(t+u) \; d_{i}(t)dt$$

quelque soit j. Cette propriété permet d'obtenir effectivement $N_F(N-M)$ fonctions d'intercorrélation indépendantes dont la moyenne est effectuée par un additionneur 320. Comme conséquence de l'impédance des fonctions de corrélation, la fonction de corrélation moyenne :

$$C(to,u-\tau) = \sum_{i,j} c_{ii}^{j}(to,u-\tau)$$

présente une variance $\sigma^2$ divisée par $N_F$.

La fonction $C(to,u-\tau)$ est finalement traitée de façon classique par un interpolateur 330 qui fournit l'estimée V de la vitesse cherchée en calculant la position uo $= \tau = 2VT/C$ du pic de la corrélation, dont la hauteur $C_{Max}$ est également mesurée en vue d'être utilisée dans un circuit de validation 340 de la valeur estimée V de la vitesse.

La validation de la mesure de vitesse est indispensable. Car, en dehors des zones d'écoulement, le signal sortant de l'éliminateur 200 d'échos fixes est essentiellement du bruit. Le résultat alors fourni par le dispositif selon l'invention n'est pas l'indication d'une vitesse nulle, et il devient nécessaire de valider, ou non, ce résultat. Pour cela, on procède à deux comparaisons. D'une part, on calcule l'énergie locale E du signal en sortie de l'éliminateur d'échos fixes :

$$E = \sum_{i=1}^{N-M+1} d_{i}^{2}(t)$$

E est ensuite comparée à un seuil Eo. Si le résultat de cette comparaison est positif, on procède alors à la comparaison du maximum $C_{Max}$ du pic de corrélation à un deuxième seuil $E_1$. L'estimée V de la vitesse ne sera validée que si $C_{Max}$ est supérieur à $E_1$.

La vitesse ainsi validée est traitée pour visualisation, de façon classique, par une unité 400, non représentée, comportant un dispositif de mémorisation, conversion de balayage et codage de couleur.

## Revendications

1. Dispositif de mesure de la vitesse d'écoulements sanguins à partir d'une séquence de N signaux échographiques successifs, comprenant un éliminateur (200) d'échos fixes à M points, suivi d'une unité de mesure (300) de ladite vitesse par corrélation-sommation-interpolation des N-M + 1 signaux indépendants issus dudit éliminateur (200) d'échos fixes, caractérisé en ce que ladite unité de mesure (300) comporte, d'une part, $N_F$ voies parallèles (310j) de traitement constituées, chacune, d'un filtre (311j), les $N_F$ filtres (311) étant complètement décorrélés entre eux, d'une mémoire (312j) de stockage des N-M + 1 signaux filtrés, et d'un corrélateur (313j) fournissant N-M + 1 fonctions d'intercorrélation, et, d'autre part, un additionneur (320) qui réalise la moyenne des $N_F(N-M)$ fonctions d'intercorrélation obtenues, un circuit (330) d'interpolation appliqué à la fonction d'intercorrélation moyenne fournissant une estimée de la vitesse recherchée.

2. Dispositif de mesure selon la revendication 1, caractérisé en ce que, Fe étant la fréquence d'échantillonnage dudit dispositif, chaque filtre (311j) est défini sur l'intervalle ]0,Fe/2] par une réponse en module égale à 1 et une phase aléatoire, et par une réponse nulle pour la fréquence zéro.

3. Dispositif de mesure selon l'une des revendications 1 ou 2, caractérisé en ce que chaque mémoire (312j) de stockage ne mémorise que le signe des N-M + 1 signaux issus du filtre (311j) correspondant.

4. Dispositif de mesure selon l'une quelconque des revendications 1 à 3, caractérisé en ce qu'il dispose d'un circuit (340) de validation de l'estimée obtenue pour ladite vitesse.

**Claims**

1. A device for measuring the speed of blood flows on the basis of a sequence of N successive echographic signals, comprising an M-point fixed-echo elimination device (200), followed by a unit (300) for measuring said speed by correlation/summation/interpolation of the N-M + 1 independent signals supplied by said fixed-echo elimination device (200), characterized in that said measuring unit (300) comprises on the one hand $N_F$ parallel processing channels (310j), each of which is formed by a filter (311j), the $N_F$ filters (311) being completely decorrelated from one another, a memory (312j) for storing the N-M + 1 filtered signals, and a correlation device (313j) which supplies N-M + 1 intercorrelation functions, and on the other hand an adder (320) which forms the mean value of the $N_F$(N-M) intercorrelation functions obtained, an estimate of the relevant speed being supplied by an interpolation circuit (330) which acts on the mean intercorrelation function.

2. A measuring device as claimed in Claim 1, characterized in that, Fe being the sampling frequency of said device, each filter (311j) is defined on the interval ]0, Fe/2] by a modulus response equal to 1 and a random phase, and by a response zero for the frequency zero.

3. A measuring device as claimed in any one of the Claims 1 or 2, characterized in that each storage memory (312j) stores only the sign of the N-M + 1 signals supplied by the corresponding filter (311j).

4. A measuring device as claimed in any one of the Claims 1 to 3, characterized in that it comprises a circuit (340) for validating the estimate obtained for said speed.

**Patentansprüche**

1. Anordnung zum Messen der Strömungsgeschwindigkeit von Blut, wobei von einer Folge von N aufeinanderfolgenden echographischen Signalen ausgegangen wird, mit einem Festechoaustaster (200), mit M Punkten, dem eine Einheit (300) zum Messen der Geschwindigkeit durch Kortelation-Summierung-Interpolation von N-M + 1 unabhängigen vom Festechoaustaster (200) ausgegebenen Signalen nachgeschaltet ist, dadurch gekennzeichnet, daß die Meßeinheit (300) einerseits $N_F$ parallelen Bearbeitungswege (310j) enthält, die je ein Filter (311j), wobei die $N_F$ Filter (311) untereinander ganz dekorreliert sind, einen Speicher (312j) zum Speichern der N-M + 1 gefilterten Signale und einen Korrelator (313j) zur Lieferung von N-M + 1 Zwischenkorrelationsfunktionen enthalten, und andererseits einen Addierer (320), der da Mittelwert der $N_F$(N-M) erhaltenen Zwischenkorrelationsfunktionen verwirklicht, und eine Interpolationsschaltung (330) enthält, die zur Erzeugung der mittleren Zwischenkorrelationsfunktion dient, wodurch eine gesuchte Geschwindigkeitsschätzung erhalten wird.

2. Meßanordnung nach Anspruch 1, dadurch gekennzeichnet, daß, wenn Fe die Abtastfrequenz der Anordnung ist, jedes Filter (311j) im Intervall ]0,Fe/2] durch eine Beantwortung mit einem Modul gleich 1 und eine zufallsbedingte Phase sowie durch eine Nullbeantwortung für die Nullfrequenz definiert wird.

3. Meßanordnung nach einem der Ansprüche 1 oder 2, dadurch gekennzeichnet, daß jeder Speicher (312j) nur das Vorzeichen der vom entsprechenden Filter (311j) ausgegebenen N-M + 1 Signale speichert.

4. Meßanordnung nach einem der Ansprüche 1 bis 3, dadurch gekennzeichnet, daß sie eine Auswertungsschaltung (340) zum Auswerten der für die Geschwindigkeit erhaltene Schätzung enthält.

FIG.1

FIG.2a

FIG.2b

7

**FIG.3**